# EUROPEAN PATENT APPLICATION

(11) **EP 4 070 749 A1**
(43) Date of publication of application: **12.10.2022**
(21) Application number: 20896358.7
(22) Date of filing: 27.11.2020
(51) Int. Cl.: A61B 18/12

(54) **COMBINED-TYPE OPERATION ADVANCING DEVICE FOR HYSTEROSCOPE INSTRUMENT**

(30) Priority: 06.12.2019 CN 201922171673 U; 15.05.2020 CN 202020812832 U; 15.05.2020 CN 202010415158
(71) Applicant: Zhang, Chongyi, Xi'an, Shaanxi 710077 (CN)
(72) Inventor: Zhang, Chongyi, Xi'an, Shaanxi 710077 (CN)
(74) Representative: Ehrner & Delmar Patentbyrå AB
(86) International application number: PCT/CN2020/132419
(87) International publication number: WO 2021/109941

(57) **Abstract**

The present invention disclosed a combined hysteroscope instrument operation propeller, which includes a propelling body that can be attached to the hysteroscope body, the instrument can smoothly pass through the blind areas such as the vagina and cervical canal along the hysteroscope body that has already entered with the help of the sheath on the propelling body, and can be operated continuously and flexibly after being sent into the uterus. The present invention is beneficial for doctors to quickly, accurately and non-invasively send the treatment instrument into the uterus, which not only reduces the treatment cost, but also achieves a good treatment effect.

## Description

### TECHNICAL FIELD

The present invention relates to a hysteroscope surgical treatment system, and more particularly to a device attached to a hysteroscope body to assist instruments to enter the uterine cavity.

### BACKGROUND

In the process of hysteroscopic treatment, the commonly used resectoscope, therapeutic mirror and planing system have in common that the inspection mirror body and multiple therapeutic instruments are wrapped together, resulting in a relatively thick system, which often needs to dilate the cervix to more than 8mm. Moreover, the instruments used for treatment operations are generally fine in structure, often expensive, complicated and time-consuming to operate, which makes it difficult to perform hysteroscopic surgery using these systems in outpatient clinics.

In the process of hysteroscopy, even if polyps, intrauterine adhesions, and submucosal fibroids are found in the uterine cavity, it is not easy due to scissors, electric cutters, electric cutting rings, electro-acupunctures, electrocoagulation rods and other therapeutic instruments alone are not easy to pass through blind spots such as vagina and cervical canal, which requires a higher skill for the doctors to further carry out hysteroscopic treatment. In addition, due to the brittle texture of the electric cutters, the electric cutting rings, the electro-acupunctures, and the electrocoagulation rods, and are easy to be damaged; and front end of the scissors is sharp, and is easy to scratch the mucosa of the vagina and cervical canal, such that the widespread outpatient hysteroscopic surgery has multiple limiting factors.

Chinese patent CN201379632Y discloses a uterine cavity surgical instrument, in which the uterine forceps and the hysteroscope can be moved synchronously and can be operated independently at the same time to form a system. It can ensure that the entry and operation of the instruments are under the monitoring of the hysteroscope, but the hysteroscope needs to be returned when the instruments need to be replaced, which leads to a long time for the entire treatment process, and the limited operating range of the instruments leads to the view of the hysteroscope frequently replacing, the continuity of the operation is reduced and the treatment effect is affected.

### TECHNICAL PROBLEM

An object of the present invention is to provide a combined hysteroscope instrument operation propeller, the structure is simple and is easy to be operated.

### SUMMARY

In order to achieve above object, the present invention adopts the technical solution as following:
The propeller includes a cylindrical propelling body, one side surface of the cylindrical propelling body is provided with an engagement groove configured for connecting with a hysteroscope body, and the other side surface of the cylindrical propelling body is provided with a guiding convex strip extending along the hysteroscope body, the guiding convex strip is provided with a sheath, and the sheath includes a sleeve body slidably matched with the guiding convex strip and a drive rod connected to the sleeve body.

In one embodiment, a transverse section (referring to the surface of the cylindrical propelling body contacting with the hysteroscope body) of the engagement groove is arc-shaped (a radian of the engagement groove is greater than π, such that the cylindrical propelling body can be firmly attached to the hysteroscope body to prevent the cylindrical propelling body from falling off).

In one embodiment, the sheath further includes a slide rail provided on the sleeve body and connected to the guiding convex strip (the part of the sleeve body except for the space occupied by the slide rail is used to accommodate the front end of the electric cutters, electric cutting rings, electro-acupunctures, electrocoagulation rods and scissors), and one end of the drive rod is arranged on an outer wall surface of the slide rail.

In one embodiment, a front end (the end close to the front end of the hysteroscope body) of the sleeve body is configured to be an arc-shaped or a bullet-shaped shape (in order to facilitate the installation of the slide rail matched with the guiding convex strip and the sliding of the sleeve body, the sleeve body retains a part of the outer circumference of the complete bullet head revolving body in shape, and the same is true for the arc-shaped sleeve body).

In one embodiment, a length of the cylindrical propelling body is less than a length of the hysteroscope body, and distances between two ends of the cylindrical propelling body and corresponding ends (especially the front end, that is the collecting end of the checking image) of the hysteroscope body are adjusted by relative sliding of the engagement groove and the hysteroscope body.

In one embodiment, a surface portion of the cylindrical propelling body located between an opening of the engagement groove and the guiding convex strip is protruded outwardly, and the protruded surface portion is divided into two sections symmetrically distributed along a longitudinal section (taking an axial direction of the hysteroscope body as the longitudinal direction) of the cylindrical propelling body, and a transverse section of an outwardly protruded surface of each section is arc-shaped (which jointly constitutes the remaining surface of the cylindrical propelling body except for the engagement groove, the guiding convex strip, and the end surface).

In one embodiment, the propeller can be used with a speculum or without a speculum; for example, the hysteroscope body, the cylindrical propelling body and the instruments can be passed through the speculum into the uterine cavity, and the colposcopy technique can also be used without using a speculum, directly into the hysteroscope body, after the hysteroscope body enters the uterine cavity, and then use the cylindrical propelling body to send the instrument into the uterine cavity.

The method for operating the propeller above includes following steps:
1) combining the cylindrical propelling body with the hysteroscope body through the engagement groove;
2) sending the hysteroscope body into a uterine cavity; and
3) adjusting a position of the cylindrical propelling body along the hysteroscope body under a hysteroscope view, such that a surgical instrument is able to move with the sheath (the sleeve body can slide along the guiding convex strip by operating the drive rod, so as to realize the movement of the sheath) and enter the uterine cavity.

### BENEFICAL EFFECTS

In the present invention, by using the engagement groove of the cylindrical propelling body, the guiding convex strip can be introduced along the hysteroscope body and fixed in the channel entering the uterine cavity, combined with the cooperative sliding of the sheath, the instrument with the front end being more easily damaged or the tissue around the channel being easily damaged can be quickly, accurately and non-invasively sent into the uterine cavity without hysteroscope monitoring, and once the front end of the instrument enters the hysteroscope monitoring area (can be withdrawn or withdrawn from the sheath), the doctor can continuously complete a surgical operation of lesions (polyps , intrauterine adhesions, submucosal fibroids in the uterine cavity) at different angles and depths (the operating range is larger and the flexibility is larger) within a certain field of view, such that the treatment process is simple and does not require to dilate the cervix or the size that need to dilate the cervix is reduced, the operation process is also safer, the treatment cost and time are reduced, and the pain degree of treatment is reduced, and the corresponding operation can be completed in the outpatient clinic. In addition, the popularization and application of the present invention can also reduce the cost of the instrument (the instrument with a simpler structure can be used, and the instrument does not need complicated and fine structural design).

Further, in the present invention, the engagement groove, the convex surface between the guiding convex strip and the engagement groove, and the outer shape of the sleeve body of the sheath are all curved surfaces without edges and corners, so that the entering process of the propeller and the instrument along the hysteroscope body is relatively stable and avoids damage to normal tissues such as the uterine cavity.

### BRIEF DESCRIPTION OF THE DRAWINGS

Fig. 1 is a front view of a hysteroscopic instrument operation propeller in an embodiment 1;
Fig. 2 is the bottom view of a hysteroscopic instrument operation propeller in an embodiment 1;
Fig. 3 is a schematic view of a transverse section (A-A) of a hysteroscopic instrument operation propeller in an embodiment 1;
Fig. 4 is a perspective view of the hysteroscopic instrument operation propeller in an embodiment 2;

In the figures:
1 hysteroscope body; 1-1 rear end of the hysteroscope body; 1-2 front end of the hysteroscope body; 2 propelling body; 2-1 convex strip; 2-2 handle; 3 sheath; 3-1 operating lever.

### DETAILED DESCRIPTION

The present invention will be described in further detail below with reference to the accompanying drawings and embodiments.

### Embodiment 1:

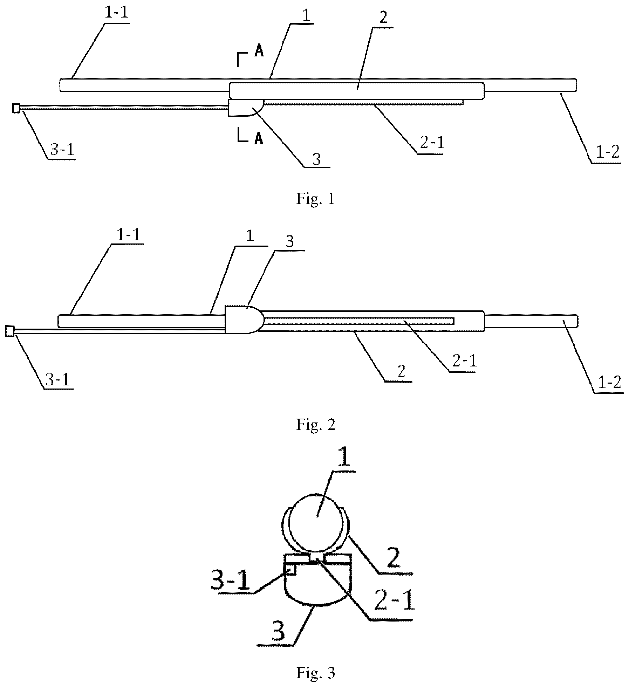

Referring to Fig. 1, Fig. 2 and Fig. 3, the hysteroscopic instrument operation propeller according to the present invention includes a propelling body 2 and a sheath 3, and one side surface of the propelling body 2 is a concave large semi-cylindrical surface, forming the engagement groove of the propelling body 2, the propelling body 2 can be reliably attached to the hysteroscope body 1 through the engagement groove, and the other side surface of the propelling body 2 (the back of the engagement groove) is provided with a convex strip 2-1. The sheath 3 has a sleeve body designed with a bullet-shaped shape, and the sleeve body is provided therein with a space configured for accommodating the sharp front end of the scissors, and easily damaged front ends of electric cutters, electric cutting rings, electro-acupunctures, electrocoagulation rods, etc., and further provided with a slide rail fixed on the inner wall of the sleeve body (the convex groove type can be used). The convex strip 2-1 is embedded in the slide rail and can slide relative to the slide rail (along the axis of the hysteroscope body). The sliding of the sleeve body is driven by an operating rod 3-1, one end of the operating rod 3-1 is fixed on the outer side wall of the slide rail (does not affect the sliding of the slide rail and the convex strip), and the other end faces the rear end of the hysteroscope body 1- 1 and extends out the sleeve body (the length is suitable for easy holding operation); the surface portion of the propelling body 2 between the opening of the engagement groove and the corresponding side of the convex strip 2-1 is an outwardly convex arc-shaped, which can reduce the interference with the surrounding tissue. The surrounding tissue can also be separated from the convex strips 2-1 to a certain extent, so as to avoid affecting the sliding process of the sleeve body to the greatest extent. The length of the propelling body 2 is about 115mm (the length of the hysteroscope body is about 200mm), and the length of the sleeve body is about 15mm, both of them can be made of stainless steel. After the propelling body 2 is engaged on the hysteroscope body 1, the distance between the front end of the propelling body 2 and the front end of the hysteroscope body can be adjusted (for example, after the propelling body 2 is observed in the hysteroscope monitoring area). The front end of the propelling body 2 can be processed into an arc-shaped to facilitate the movement of the propelling body 2 (for example, to reduce hooking and dragging with surrounding tissues), and which can also reduce the shielding of the hysteroscope monitoring area (especially when the front end of the propelling body 2 approaches the front end 1-2 of the hysteroscope body or overlaps with the front end 1-2 of the hysteroscope body), the sheath 3 sliding on the convex strip 2-1 can be positioned accurately, so as to fully play a protective role in the movement of the instrument.

During hysteroscopic surgery, the propelling body 2 is firstly installed (pressed or inserted) on the rear end 1-1 of the hysteroscope body, then the hysteroscope body 1 is sent into the uterus. During the hysteroscopy process, when polyps, intrauterine adhesions, submucosal fibroids, etc. are found in the uterine cavity, the instrument can be sent for treatment. At this time, according to the width of the internal orifice of the cervical canal, the front end 1-2 of the hysteroscope body is kept at the uterine cavity or the internal orifice of the cervical canal, and the front end of the propelling body 2 is pushed into the front end 1-2 of the hysteroscope body and stop until the front end of the propelling body 2 can be seen on the display of the hysteroscope, and the propelling body 2 can be pushed or pulled slightly to adjust the position of the front end of the propelling body 2 (to ensure that the field of view of the hysteroscope monitoring area on the display is always good before the sheath 3 enters and after the instrument enters), the sleeve body of the sheath 3 is connected to the convex strip 2-1 (through the slide rail), and then the front end of the scissors, electro-acupunctures, electro-cutting rings and other instruments of the laparoscope are put into the sleeve body (whichever instrument needs to be used, put it into the sleeve body), the sleeve body is pushed to the front end 1-2 of the hysteroscope body using the operating lever 3-1(push the instrument together), until the tail of the sleeve body is seen on the display of the hysteroscope. The scissors or electro-acupunctures or electric cutting rings and other instruments are removed from the sleeve body under the direct view of the hysteroscope, and when the normal treatment operations can be performed. When it is necessary to replace the instruments for the treatment operation, withdraw the instruments currently used for the operation (if the instruments need to be replaced during the treatment, such as scissors, they can be taken out directly after they are folded, the instruments such as electro-acupuncture, electric cutting rings, etc. that are not easy to take out, which can be put into the sleeve body and take it out together with the sheath), and then repeat the above method of sending the instrument, and then the new treatment operation can be continued.

Since polyps, intrauterine adhesions, and submucosal fibroids are found in the uterine cavity during the hysteroscopy, it was necessary to enter the treatment instruments. By using the above propeller, when the instruments pass through the blind areas of the vagina and cervical canal, which can be smoothly sent into (and withdrawn from) the uterus along the hysteroscope body 1 that has already entered, therefore, the interval time between treatment operations can be shortened, and since the device integrating the hysteroscope body and the instrument is no longer required, the cervix is required to be about 6mm, so that some conventional treatments basically do not need to dilate of the cervix, which can reduce the intraoperative pain of the patient and avoid hospitalization. Furthermore, the operation of the instrument is less restricted, and it can be operated continuously and flexibly under the hysteroscopic field of view provided by the display. In addition, it can also meet the objective requirements for the durability and easy availability of the treatment instrument, and selecting to use the treatment instrument with the structure being simpler and the size being larger (which makes the instrument easier to handle and less prone to damage).

### Embodiment 2:

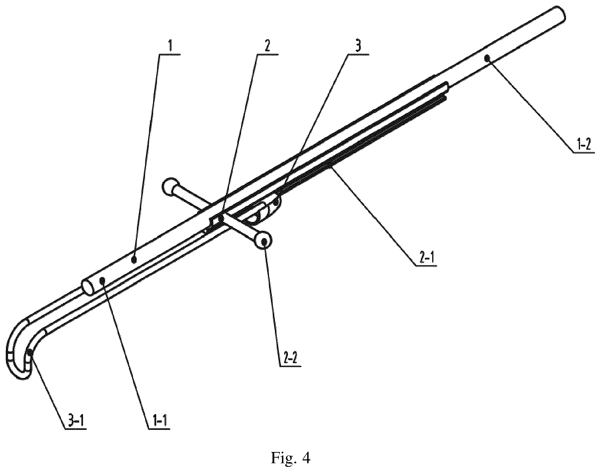

Referring to Fig. 4, the difference from the embodiment 1 is that the operating rod 3-1 is increased from one to two, and connected to a U-shaped structure, and the fixed position of the operating rods 3-1 at the slide rail are adjusted from the lower side to the upper side, in addition, a handle 2-2 is added to the propelling body 2, so that the operation of the propeller is more convenient.

In a word, the hysteroscopic instrument operation propeller of the present invention is beneficial for doctors to quickly, accurately and non-invasively send the treatment instrument into the uterus, and the treatment process can be completed in the outpatient operating room, which not only reduces the treatment cost, but also achieves a good treatment effect.

## Claims

1. A combined hysteroscopic instrument operation propeller, comprising:
a cylindrical propelling body(2), one side surface of the cylindrical propelling body (2) being provided with an engagement groove configured for connecting with a hysteroscope body (1), and the other side surface of the cylindrical propelling body (2) being provided with a guiding convex strip (2-1) extending along the hysteroscope body (1), wherein the guiding convex strip (2-1) is provided with a sheath (3), and the sheath (3) comprises a sleeve body slidably matched with the guiding convex strip (2-1) and a drive rod connected to the sleeve body.

2. The combined hysteroscopic instrument operation propeller according to claim 1, wherein a transverse section of the engagement groove is arc-shaped.

3. The combined hysteroscopic instrument operation propeller according to claim 2, wherein a radian of the engagement groove is greater than π.

4. The combined hysteroscopic instrument operation propeller according to claim 1, wherein the sheath (3) further comprises a slide rail provided on the sleeve body and connected to the guiding convex strip (2-1).

5. The combined hysteroscopic instrument operation propeller according to claim 4, wherein one end of the drive rod is arranged on an outer wall surface of the slide rail.

6. The combined hysteroscopic instrument operation propeller according to claim 1, wherein the sleeve body is configured to be an arc-shaped or a bullet-shaped shape.

7. The combined hysteroscopic instrument operation propeller according to claim 1, wherein a length of the cylindrical propelling body (2) is less than a length of the hysteroscope body (1), and distances between two ends of the cylindrical propelling body (2) and corresponding ends of the hysteroscope body (1) are adjusted by relative sliding of the engagement groove and the hysteroscope body (1).

8. The combined hysteroscopic instrument operation propeller according to claim 1, wherein a surface portion of the cylindrical propelling body (2) located between an opening of the engagement groove and the guiding convex strip(2-1) is protruded outwardly, and the protruded surface portion is divided into two sections symmetrically distributed along a longitudinal section of the cylindrical propelling body (2), and a transverse section of an outwardly protruded surface of each section is arc-shaped.

9. A method for operating the combined hysteroscope instrument operation propeller according to claim 1, comprising following steps:
1) combining the cylindrical propelling body (2) with the hysteroscope body (1) through the engagement groove;
2) sending the hysteroscope body (2) into a uterine cavity; and
3) adjusting a position of the cylindrical propelling body (2) along the hysteroscope body (1) under a hysteroscope view, such that a surgical instrument is able to move with the sheath (3) and enter the uterine cavity.
